# EUROPEAN PATENT SPECIFICATION

(11) **EP 2 040 858 B1**
(45) Date of publication and mention of the grant of the patent: **15.02.2012**
(21) Application number: 07720222.4
(22) Date of filing: 06.07.2007
(51) Int. Cl.: B05C 17/01, B05C 17/005

(54) **DISPENSING APPLIANCE FOR A MULTIPLE CARTRIDGE**
ABGABEVORRICHTUNG FÜR EINE MEHRFACHPATRONE
DISPOSITIF DE DISTRIBUTION POUR UNE CARTOUCHE MULTIPLE

(30) Priority: 17.07.2006 CH 11482006
(43) Date of publication of application: 01.04.2009
(73) Proprietor: Medmix Systems AG, 6343 Rotkreuz (CH)
(72) Inventor: KELLER, Wilhelm A., CH-6402 Merlischachen (CH)
(74) Representative: Detken, Andreas
(86) International application number: PCT/CH2007/000330
(87) International publication number: WO 2008/009143

(56) References cited:
- EP-A- 0 941 940
- EP-A1- 0 621 083
- WO-A2-2004/067962
- DE-A1- 10 128 611
- FR-A- 1 179 121
- US-A- 1 569 268
- US-A- 3 166 221
- US-A- 3 952 920
- US-A- 4 623 337
- US-A- 5 535 922
- US-A1- 2002 166 878
- US-A1- 2003 089 743

## Description

The present invention relates to a dispensing assembly comprising a dispensing appliance and a multiple cartridge, comprising a housing for receiving the cartridge that has a thread and a rotatable portion that has a complementary thread, according to the preamble of claim 1.

A dispensing appliance of this kind is known from DE-101 28 611 A1, the appliance having two concentrically arranged storage containers and the rotatable portion comprising a ram with an inner and an outer ram part, the outer ram part being provided with the thread.

CH-322 201 also discloses a dispensing appliance where the storage containers are arranged concentrically and one of the rods of the dispensing pistons is provided with a threaded portion that cooperates with a corresponding threaded portion in the center of the inner storage cylinder.

There exist further a wealth of dispensers where one part of the housing rotates in relation to another part for extruding pharmaceutical and the like materials, such as US 2003/0089743 A1, US 2002/0166878 A1, DE 101 28 611 A1, and US-Patent No. 4,623,337. However, all those prior art documents disclose a single component cartridge or syringe, where the problem adressed is much simpler.

Another category are dispensers with a threaded dispensing movement and two adjacent storage cylinders which are disclosed in US-Patent No. 3, 952,920 and EP 0 621 083 A1, where a central threaded bar bears on rams. These solutions are not adapted for medical use and are very difficult to sterilize.

Furthermore, manually operated dispensing appliances for a double cartridge for dispensing two-component materials are available on the market which are designed in the manner of a gun, for example US-Patent No. 5,535,922. During the return movement of the lever transmission mechanism, the double ram is relieved and a total pressure relief of the cartridge is caused, thereby interrupting the material flow. An accurately metered application as it is advantageous particularly in the application of bone cements is thus impossible. Furthermore, in the case of highly viscous materials, limits are imposed on such appliances by the fact that either a high multiplication ratio is required and thus a complicated mechanism, or high forces have to be applied.

On the background of this prior art, it is an object of the present invention to provide a screw feed dispensing appliance that is suitable for multiple cartridges or multiple syringes having at least two adjacent storage containers, whose construction is simple, that allows even highly viscous materials to be dispensed with a relatively low manual force expenditure and improved accuracy, and is suitable for medical applications.

The invention will be explained in more detail hereinafter with reference to drawings of an exemplary embodiment.
- Fig. 1: shows a perspective view of the dispensing appliance according to the invention,
- Fig. 2: shows an exploded view of the dispensing appliance of Fig. 1 as well as the individual elements of a double cartridge,
- Fig. 3: shows a longitudinal section of the appliance of Fig. 1, and
- Fig. 4: shows a section according to line IV-IV in Fig. 3.

As appears in the drawings, the principle of the dispensing appliance according to the invention is based on mutual interpenetration of two parts that are rotated with respect to one another, whereby a continuous and accurately metered feed can be achieved. Hereinafter, the term "double cartridge" in the exemplary embodiment stands for a multiple cartridge or syringe. In this regard, it is apparent to one skilled in the art that in the case of more than two storage containers, a corresponding number of pistons and rams will have to be provided.

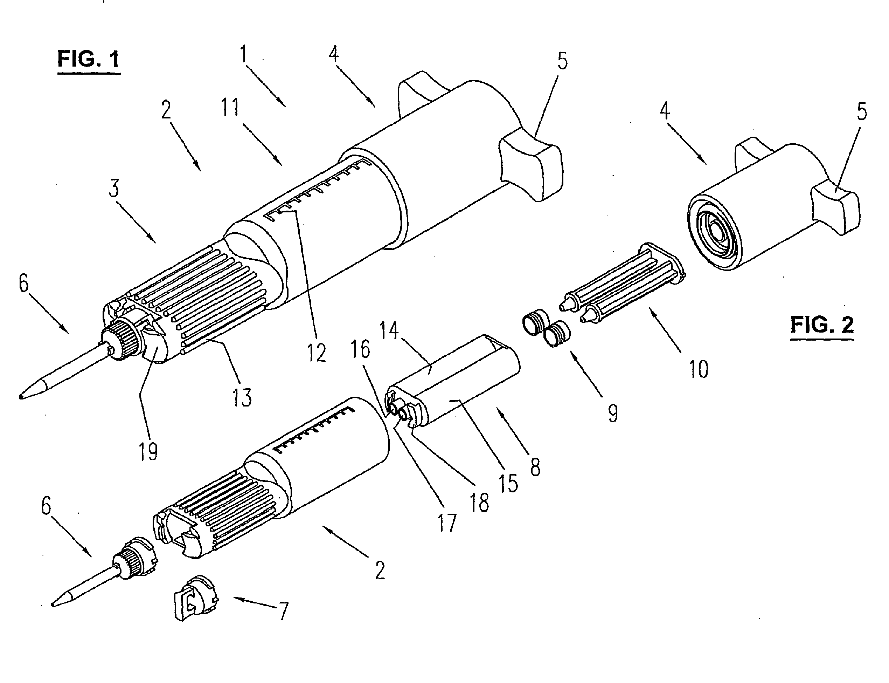

Fig. 1 shows dispensing appliance 1 with housing 2 and gripping area 3 thereof as well as a rotatable portion 4 provided with rotary lobe 5. These parts are also found in Fig. 2, where a conventional mixer 6 and a conventional closure cap 7 are shown at the dispensing end. Between housing 2 and rotatable portion 4, a double cartridge 8 with pistons 9 and double ram 10 is arranged.

Furthermore, in Figs. 1 and 2, a graduation 12 is applied on housing section 11 on the inlet side of housing 2 and ridges 13 are provided on gripping area 3 of housing 2 in order to ensure a rotationally secure grip.

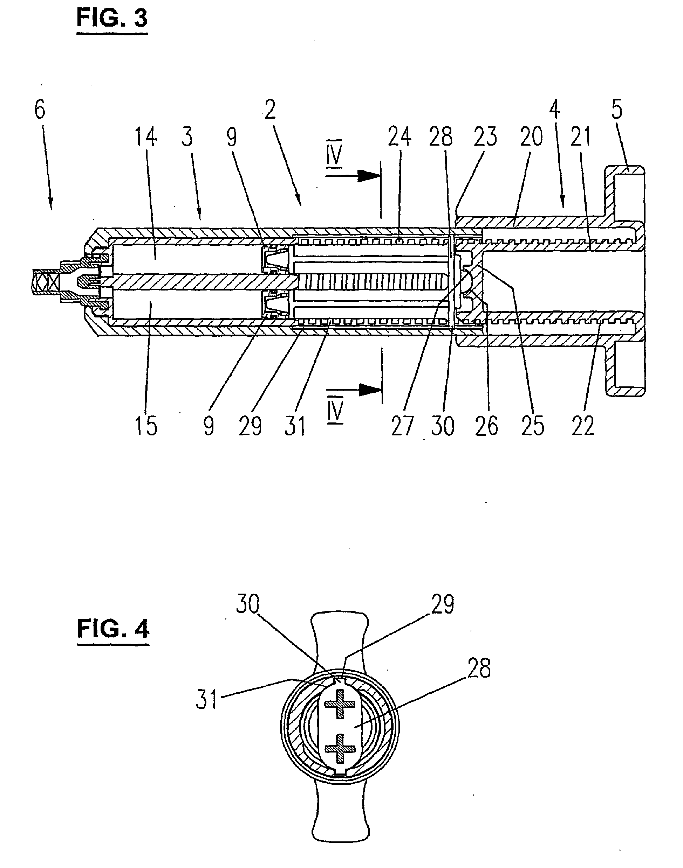

Cartridge 8 has two adjacent storage cylinders 14 and 15 having each the same diameter. However, the storage cylinders can also have different diameters or volumes, respectively. The cartridge further comprises two outlets 16 and 17 as well as bayonet sockets 18. As appears in Figs. 1 and 3, bayonet sockets 18 come to rest on correspondingly shaped bayonet reinforcement members 19 on the gripping area of housing 2.

In Fig. 3 it is apparent that rotatable portion 4 is essentially composed of an internal cylinder 21 having an external thread 22 and of a cylindrical outer sleeve 20. End portion 23 on the outlet side of sleeve 20 serves as an indicator edge that cooperates with graduation 12 on housing 2 for indicating the dispensed volume.

In Fig. 3 it is further visible that housing section 11 of housing 2 is provided with an internal thread 24 that cooperates with external thread 22 on internal cylinder 21 of the rotatable portion. The force transmission from the rotatable portion to the double ram is achieved by a bearing 25 on the dispensing side of rotatable portion 4 that has a centrally arranged, spherically shaped bearing surface 26 which cooperates with a corresponding spherically shaped bearing surface 27 in the center of thrust plate 28 of double ram 10. This arrangement ensures that only a small torque is transmitted to the thrust plate. This torque is absorbed by two guiding grooves 29 in housing section 11, see Fig. 4, in which two corresponding cams 30 on thrust plate 28 are guided. In order to allow the displacement of thrust plate 28, the internal thread has two opposed passages in the form of grooves 31.

In Fig. 3, the elements of Fig. 2 are shown while the appliance is ready for dispensing. The force transmission by a thread allows dispensing highly viscous materials without a great force expenditure and mainly also avoiding a complete pressure relief of the rams and thus an interruption of the material flow.

## Claims

1. Dispensing assembly comprising a dispensing appliance and a cartridge or syringe, the dispensing appliance comprising a housing (2) for receiving the cartridge or syringe (8), the housing having a thread and a rotatable portion having a complementary thread, the two parts cooperating in such a manner that by mutual rotation thereof, the rotatable portion is continuously displaceable relative to the housing in the dispensing direction, **characterised in that** the housing (2) receives said cartridge or syringe (8) , said cartridge being a multiple cartridge or syringe (8) and having at least two adjacent storage containers (14, 25) and a multiple ram (10) and that the thrust force of the rotatable portion (4) is transmitted to the multiple ram (10) sliding in the storage containers (14, 15) of the multiple cartridge or syringe, whereby the rotatable portion (4) is provided on its outlet side with a centrally arranged bearing (25) acting upon a thrust plate (28) of the multiple ram (10), the thrust plate (28) being non-rotatably guided inside the housing.

2. Dispensing assembly according to claim 1, **characterised in that** the rotatable portion (4) is essentially composed of an external sleeve (20) and of an internal cylinder (21) having an external thread (22), the external thread (22) cooperating with a corresponding internal thread (24) in the housing (2).

3. Dispensing assembly according to claim 1, **characterised in that** the bearing (25) on the rotatable portion (4) has a spherically shaped bearing surface (26) that corresponds to a correspondingly spherically shaped bearing surface (27) on the thrust plate.

4. Dispensing assembly according to claim 1 or 3, **characterised in that** the thrust plate (28) has two cams (30) that are guided in respective guiding grooves (29) inside the housing.

5. Dispensing assembly according to any one of claims 1 to 4, **characterised in that** the multiple cartridge or syringe is provided at its outlet end with bayonet slots (18) that come to rest on bayonet reinforcement members (19) at the outlet end of the housing.

6. Dispensing assembly according to any one of claims 1 to 5, **characterised in that** the housing (2) of the multiple cartridge or syringe is provided with a graduation (12) that cooperates with the front edge (23) of the sleeve (20) of the rotatable portion (4) in order to indicate the dispensed volume.

## Patentansprüche

1. Austraganordnung, aufweisend ein Austragvorrichtung und eine Kartusche oder Spritze, wobei die Austragvorrichtung ein Gehäuse (2) aufweist, um die Kartusche oder Spritze (8) aufzunehmen, wobei das Gehäuse ein Gewinde und einen drehbaren Bereich mit einem dazu komplementären Gewinde aufweist, wobei diese beiden Teile auf eine solche Weise zusammenwirken, dass bei einer Drehung der Teile relativ zueinander der drehbare Bereich gegenüber dem Gehäuse kontinuierlich in die Austragrichtung verschiebbar ist, **dadurch gekennzeichnet, dass** das Gehäuse (2) die Kartusche oder Spritze (8) aufnimmt, wobei die Kartusche eine Mehrfachkartusche oder -spritze (8) ist und mindestens zwei nebeneinanderliegende Lagerbehälter (14, 25) und einen Mehrfachstössel (10) aufweist, und dass die Vorschubkraft des drehbaren Bereichs (4) auf den Mehrfachstössel (10), der in den Lagerbehältern (14, 15) der Mehrfachkartusche oder -spritze gleitet, übertragen wird, wobei der drehbare Bereich (4) auf seiner Auslassseite mit einem zentral angeordneten Lager (25) versehen ist, welches auf eine Vorschubplatte (28) des Mehrfachstössels (10) wirkt, wobei die Vorschubplatte (28) drehfest im Inneren des Gehäuses geführt ist.

2. Austraganordnung nach Anspruch 1, **dadurch gekennzeichnet, dass** der drehbare Bereich (4) im Wesentlichen aus einer externen Hülse (20) und einem internen Zylinder (21) mit einem Aussengewinde (22) zusammengesetzt ist, wobei das Aussengewinde (22) mit einem entsprechenden Innengewinde (24) im Gehäuse (2) zusammenwirkt.

3. Austragvorrichtung nach Anspruch 1, **dadurch gekennzeichnet, dass** das Lager (25) auf dem drehbaren Bereich (4) eine sphärisch geformte Lagerfläche (26) aufweist, die einer entsprechend sphärisch geformten Lagerfläche (27) auf der Vorschubplatte entspricht.

4. Austraganordnung nach Anspruch 1 oder 3, **dadurch gekennzeichnet, dass** die Vorschubplatte (28) zwei Nocken (30) aufweist, die in entsprechenden Führungsnuten (29) im Inneren des Gehäuses geführt sind.

5. Austraganordnung nach einem der Ansprüche 1 bis 4, **dadurch gekennzeichnet, dass** die Mehrfachkartusche oder -spritze an ihrem Auslassende mit Bajonettschlitzen (18) versehen ist, welche auf Bajonettverstärkungselementen (19) am Auslassende des Gehäuses zu liegen kommen.

6. Austraganordnung nach einem der Ansprüche 1 bis 5, **dadurch gekennzeichnet, dass** das Gehäuse (2) der Mehrfachkartusche oder -spritze mit einer Skala (12) versehen ist, die mit der Vorderkante (23) der Hülse (20) des drehbaren Bereichs (4) zusammenwirkt, um das ausgetragene Volumen anzuzeigen.

## Revendications

1. Ensemble de distribution comprenant un dispositif de distribution et une cartouche ou seringue, le dispositif de distribution comprenant un boîtier (2) destiné à recevoir la cartouche ou seringue (8), le boîtier ayant un filet et une partie rotative ayant un filet complémentaire, les deux pièces coopérant de telle sorte que grâce à la rotation mutuelle de celles-ci, la partie rotative puisse être déplacée de manière continué, par rapport au boîtier dans la direction de distribution, **caractérisé en ce que** le boîtier (2) reçoit, ladite cartouche ou seringue (8), ladite cartouche étant une cartouche ou seringue multiple (8) et ayant au moins deux récipients de stockage adjacents (14, 25) et un poussoir multiple (10), et **en ce que** la force de poussée de la partie rotative (4) est transmise au poussoir multiple (10) coulissant dans les récipients de stockage (14, 15) de la cartouche ou seringue multiple, la partie rotative (4) étant munie du côté de sa sortie d'un élément d'appui (25) disposé
centralement agissant sur une plaque de poussée (28) du poussoir multiple (10), la plaque de poussée (28) étant guidée de manière non rotative à l'intérieur du boîtier.

2. Ensemble de distribution selon la revendication 1, **caractérisé en ce que** la partie rotative (4) est essentiellement constituée d'un manchon extérieur (20) et d'un cylindre intérieur (21) ayant un filet extérieur (22), le filet extérieur (22) coopérant avec un filet intérieur (24) correspondant dans le boîtier (2).

3. Ensemble de distribution selon la revendication 1, **caractérisé en ce que** l'élément d'appui (25) sur la partie rotative (4) possède une surface d'appui de forme sphérique (26) qui correspond à une surface d'appui de forme sphérique (27) correspondante sur la plaque de poussée.

4. Ensemble de distribution selon la revendication 1 ou 3, **caractérisé en ce que** la plaque de poussée (28) possède deux saillies (30) qui sont guidées dans des rainures de guidage (29) respectives à l'intérieur du boîtier.

5. Ensemble de distribution selon l'une quelconque des revendications 1 à 4, **caractérisé en ce que** la cartouche ou seringue multiple est munie à son extrémité de sortie de fentes à baïonnette (18) qui viennent reposer sur des éléments de renforcement à baïonnette (19) au niveau de l'extrémité de sortie du boîtier.

6. Ensemble de distribution selon l'une quelconque des revendications 1 à 5, **caractérisé en ce que** le boîtier (2) de la cartouche ou seringue multiple est muni d'une graduation (12) qui coopère avec le bord avant (23) du manchon (20) de la partie rotative (4) afin d'indiquer le volume distribué.
